# EUROPEAN PATENT APPLICATION

(11) **EP 1 388 537 A1**
(43) Date of publication of application: **11.02.2004**
(21) Application number: 02724787.3
(22) Date of filing: 14.05.2002
(51) Int. Cl.: C07D 209/16, C07D 209/20, C07K 5/06, A61K 31/4045, A61K 31/405, A61K 38/05, A61K 38/06, A61P 3/04, A61P 43/00

(54) **ARGININE DERIVATIVES**

(30) Priority: 15.05.2001 JP 2001144659
(71) Applicant: TAISHO PHARMACEUTICAL CO., LTD, Tokyo 170-8633 (JP)
(72) Inventor: NAKAZATO, Atsuro, c/o TAISHO PHARMACEUTICAL, Toshima-ku, Tokyo 170-8633 (JP); OKUBO, Taketoshi, c/o TAISHO PHARMACEUTICAL, Toshima-ku, Tokyo 170-8633 (JP); UMEMIYA, Hiroki, c/o TAISHO PHARMACEUTICAL, Toshima-ku, Tokyo 170-8633 (JP)
(74) Representative: HOFFMANN EITLE
(86) International application number: PCT/JP2002/004666
(87) International publication number: WO 2002/092566

(57) **Abstract**

An arginine derivative represented by the formula: 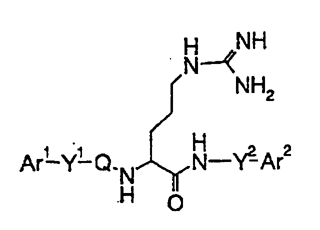 [wherein Ar¹ and Ar² may be the same or different, and are each a phenyl group, a substituted phenyl group, a naphthyl group, a substituted naphthyl group or a heteroaromatic ring group containing one or more of nitrogen, oxygen and sulfur atoms; Y¹ is a C₁₋₅ alkylene group, a C₂₋₅ alkenylene group or a single bond; and the C₁₋₅ alkylene group optionally contains a carbon atom substituted with a phenyl group, a substituted phenyl group, a naphthyl group, a substituted naphthyl group or a C₁₋₁₀ acylamino group; Q is a carbonyl group or a sulfonyl group; Y² is a C₁₋₅ alkylene group; the C₁₋₅ alkylene group optionally contains a carbon atom substituted with a phenyl group, a substituted phenyl group, a naphthyl group, a substituted naphthyl group, a hydroxyl group, a carbamoyl group, a mono-C₁₋₅ alkylamide group or a di-C₁₋₅ alkylamide group], or a pharmaceutically acceptable salt thereof.

There are provided peptidergic ligands which have the affinity and specificity to MC₄ receptor.

## Description

### TECHNICAL FIELD

The present invention relates to novel arginine derivatives which are ligands for MC₄ receptor.

### BACKGROUND ART

Melanocortins (α-, β- and γ-MSH's, and ACTH) are reported to be biosynthesized in the brain from the processing of POMC, their precursors, and to be pertinent to various physiological functions (Nature, 278, 423, 1979). Melanocortins generate the physiological functions by binding with the specific receptor. Currently, melanocortin receptors (MC receptors) are classified into 5 subtypes of MC₁ - MC₅. Among these receptors, MC₄ receptor is recognized to appear specifically in the brain, and to be widely distributed in the brain (J. Biol. Chem., 268, 15174, 1993; Mol. Endocrinol., 8, 1298, 1994).

Recently, the relation among MC₄ receptor, the appetite and the obesity has been suggested. It has been reported that, in the animal tests using selective peptidergic agonists and antagonists for MC₄ and MC₃ receptors, a strong anorectic action was observed in fast mice and various obesity models (Nature, 385, 165, 1997).

In addition, remarkable increases in the body weight, the blood insulin content and the glucose content have been observed in MC₄ receptor KO mice (Cell, 88, 131, 1997), and it was suggested that MC₄ receptor acts to control the feeding behavior and the obesity.

On the other hand, it is recognized that MC₄ receptor is also widely distributed in the limbic system (e.g., the hippocampus and amygdaloid body) and the raphe nuclei which is the origin nuclei of the serotonin nerve as well as the hypothalamus which is deeply pertinent to feeding behavior (Mol. Endocrinol., 8, 1298, 1994). It has further been recognized in the animal tests that ACTH and α-MSH act to body temperature regulation (Brain Res., 18, 473, 1987), to blood pressure (Am. J. Physiol., 257, R681, 1989), to neuroendocrine system (Life Sci., 25, 1791, 1979), to learning/memory (Neurosci. Biobehav. Rev., 4, 9, 1980) and to awaking (Neurosci. Biobehav. Rev., 4, 9, 1980), and reported to cause anxiety-like symptom and the activation of hypothalamus-pituitary-adrenal system (Pharmacol. Biochem. Behav., 36, 631, 1990; Peptides, 17, 171, 1996; ibid. 11, 647, 1990; ibid. 11, 915, 1990; Pharmacol. Biochem. Behav., 12, 711, 1980). However, ACTH and α-MSH are subtype non-specific agonists, and the relation of melanocortin receptor subtypes and these physiological functions has not yet been clarified.

Peptidergic agonists and antagonists have been reported to MC₄ receptor (Nature, 385, 165, 1997), but they also have the affinity to MC₃ receptor, and cannot be used for MC₄ receptor as a selective ligand. In addition, specific ligands to MC₄ receptor have not been reported at all. Accordingly, among MC receptors, there has not yet been clarified the physiological function via MC₄ receptor which appears specifically in the brain and is widely distributed in the brain.

An object of the present invention is to provide peptidergic ligands which have the affinity and specificity to MC₄ receptor and are useful as medicines.

### DISCLOSURE OF THE INVENTION

As a results of an extensive research on arginine derivatives, the present inventors have found arginine derivatives which are ligands having the affinity to MC₄ receptor, and thereby the present invention has been accomplished.

The present invention is illustrated below. The present invention is directed to an arginine derivative represented by the following formula [1]: 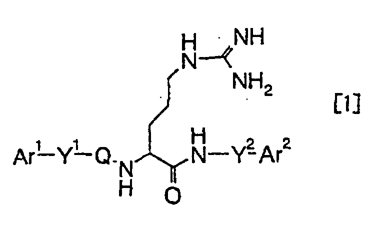 [wherein Ar¹ and Ar² may be the same or different, and are each a phenyl group, a substituted phenyl group, a naphthyl group, a substituted naphthyl group or a heteroaromatic ring group containing one or more of nitrogen, oxygen and sulfur atoms; Y¹ is a C₁₋₅ alkylene group, a C₂₋₅ alkenylene group or a single bond; and the C₁₋₅ alkylene group optionally contains a carbon atom substituted with a phenyl group, a substituted phenyl group, a naphthyl group, a substituted naphthyl group or a C₁₋₁₀ acylamino group; Q is a carbonyl group or a sulfonyl group; Y² is a C₁₋₅ alkylene group; the C₁₋₅ alkylene group optionally contains a carbon atom substituted with a phenyl group, a substituted phenyl group, a naphthyl group, a substituted naphthyl group, a hydroxyl group, a carbamoyl group, a mono-C₁₋₅ alkylamide group or a di-C₁₋₅ alkylamide group], or a pharmaceutically acceptable salt thereof. Stereoisomers exist in the arginine derivatives of the present invention, and they are also included in the present invention.

In the present invention, the substituted phenyl group refers to a phenyl group substituted with 1 to 3 substituents arbitrarily selected from the group consisting of a C₁₋₅ alkyl group, a C₁₋₅ alkoxy group, an aralkyloxy group, a hydroxyl group, a halogen atom, a nitro group, an amino group, a mono-C₁₋₅ alkylamino group, a di-C₁₋₅ alkylamino group, a trifluoromethyl group and a phenyl group; and examples of which are a 2-methylphenyl group, a 3-methylphenyl group, a 4-methylphenyl group, a 2-ethylphenyl group, a 3-ethylphenyl group, a 4-ethylphenyl group, a 2-propylphenyl group, a 3-propylphenyl group, a 4-propylphenyl group, a 2-cyclopentylphenyl group, a 2-methoxyphenyl group, a 3-methoxyphenyl group, a 4-methoxyphenyl group, a 4-ethoxyphenyl group, a 4-isopropoxyphenyl group, a 4-benzyloxyphenyl group, a 4-hydroxyphenyl group, a 2-fluorophenyl group, a 3-fluorophenyl group, a 4-fluorophenyl group, a 2-chlorophenyl group, a 3-chlorophenyl group, a 4-chlorophenyl group, a 2-bromophenyl group, a 3-bromophenyl group, a 4-bromophenyl group, a 4-nitrophenyl group, a 4-trifluoromethylphenyl group and a 4-biphenyl group.

The substituted naphthyl group refers to a naphthyl group substituted with 1 to 3 substituents arbitrarily selected from the group consisting of a C₁₋₅ alkyl group, a C₁₋₅ alkoxy group, an aralkyloxy group, a halogen atom, a nitro group, an amino group, a mono-C₁₋₅ alkylamino group, a di-C₁₋₅ alkylamino group, a trifluoromethyl group and a phenyl group; and an example of which is a 5-dimethylaminonaphthyl group.

The heteroaromatic ring group containing one or more of nitrogen, oxygen and sulfur atoms refers to a monocyclic or dicyclic aromatic ring group which contains one or more of nitrogen, oxygen and sulfur atoms; and examples of which are a 2-pyridyl group, a 3-pyridyl group, a 4-pyridyl group, a 3-indolyl group, a 3-benzothienyl group and a 4-imidazolyl group.

The C₁₋₁₀ acylamino group refers to an amino group substituted with an aliphatic or aromatic acyl group having 1 to 10 carbon atoms; and examples of which are a formylamino group, an acetylamino group, a propionylamino group, a butyrylamino group, an isobutyrylamino group, a valerylamino group, an isovalerylamino group, a pivaloylamino group, a cyclohexylamino group, a benzyloxycarbonylamino group and a t-butoxycarbonylamino group.

The C₁₋₅ alkyl group refers to a straight, branched or cyclic alkyl group having 1 to 5 carbon atoms; and examples which are a methyl group, an ethyl group, a propyl group, an isopropyl group, a cyclopropyl group, a butyl group, an isobutyl group, a cyclobutyl group, a cyclopropylmethyl group, a pentyl group, an isopentyl group, a cyclopentyl group, a cyclobutylmethyl group and a 1-ethylpropyl group.

The C₁₋₅ alkoxy group refers to a straight, branced or cyclic alkoxy group having 1 to 5 carbon atoms; and examples of which are a methoxy group, an ethoxy group, a propoxy group, an isopropoxy group, a butoxy group, an isobutoxy group, a cyclopropylmethoxy group, a pentyloxy group and an isopentyloxy group.

The mono-C₁₋₅ alkylamino group or a di-C₁₋₅ alkylamino group refers to an amino group substituted with the above-mentioned C₁₋₅ alkyl group; and examples of which are a methylamino group, an ethylamino group, a propylamino group, a dimethylamino group, a diethylamino group and a dipropylamino group.

The halogen atom refers to a fluorine atom, a chlorine atom, a bromine atom or an iodine atom.

The pharmaceutically acceptable salt refers to a salt with a mineral acid or an organic acid; and examples of which are acetate, propionate, butyrate, formate, trifluoroacetate, maleate, tartrate, citrate, stearate, succinate, ethylsuccinate, lactobionate, gluconate, glucoheptonate, benzoate, methanesulfonate, ethanesulfonate, 2-hydroxyethanesulfonate, benzenesulfonate, p-toluenesulfonate, laurylsulfate, malate, aspartate, glutaminate, adipate, cysteine salt, N-acetylcysteine salt, hydrochloride, hydrobromide, phosphate, sulfate, hydroiodide, nicotinate, oxalate, picrate, thiocyanate, undecanoate, polyacrylate salt and carboxyvinyl polymer salt.

Preferable compounds of the present invention are arginine derivatives of Formula [1] wherein Ar¹ and Ar² may be the same or different, and are each a phenyl group, a naphthyl group or a 3-benzothienyl group, Y¹ is a C₁₋₂ alkylene group or a C₁₋₂ alkylene group substituted with one acetoamino group; Q is a carbonyl group or a sulfonyl group; Y² is a C₁₋₂ alkylene group or a C₁₋₂ alkylene group substituted with one carbamoyl group, or a pharmaceutically acceptable salt thereof. More preferable are the following compounds a - n, or pharmaceutically acceptable salts thereof.
a. N²-[N-Acetyl-3-(2-naphthyl)-D-alanyl]-L-arginyl-3-(2-naphthyl)-D-alaninamide 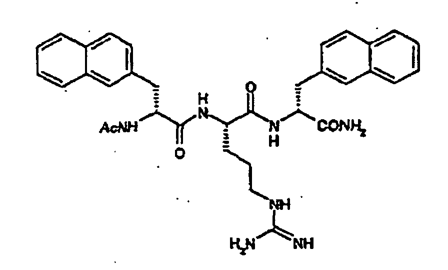
b. N²-[N-Acetyl-3-(2-naphthyl)-D-alanyl]-L-arginyl-3-(2-naphthyl)-L-alaninamide 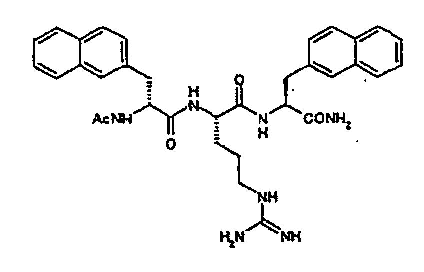
c. N²-[N-Acetyl-3-(2-naphthyl)-D-alanyl]-N-[2-(2-naphthyl)ethyl]-L-argininamide 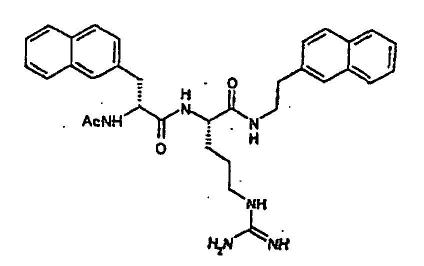
d. N²-[N-Acetyl-3-(1-naphthyl)-D-alanyl]-L-arginyl-3-(2-naphthyl)-D-alaninamide 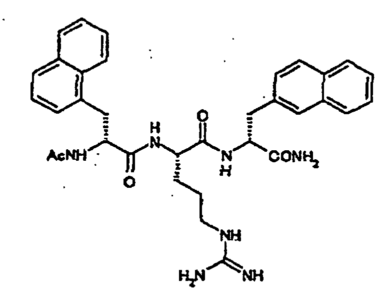
e. N²-[N-Acetyl-3-(1-naphthyl)-D-alanyl]-L-arginyl-3-(2-naphthyl)-L-alaninamide 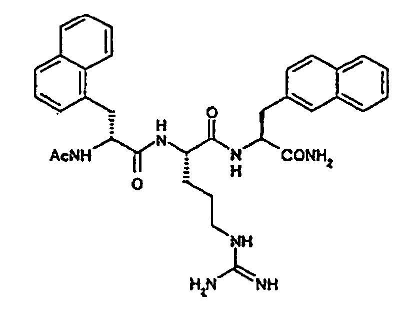
f. N²-[N-Acetyl-3-(1-naphthyl)-D-alanyl]-L-arginyl-3-(1-naphthyl)-L-alaninamide 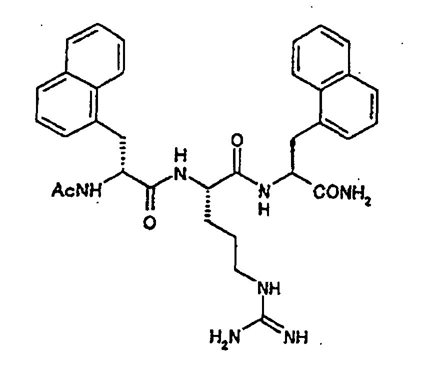
g. N²-[N-Acetyl-D-phenylalanyl]-L-arginyl-3-(2-naphthyl)-D-alaninamide 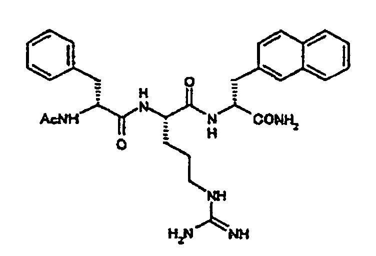
h. N²-[N-Acetyl-D-phenylalanyl]-L-arginyl-3-(2-naphthyl)-L-alaninamide 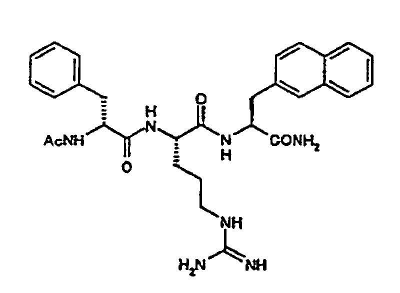
i. N²-[N-Acetyl-D-phenylalanyl]-N-[2-(2-naphthyl)ethyl]-L-argininamide 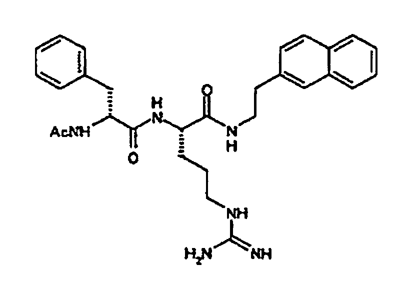
j. N²-[3-(2-Naphthyl)propionyl]-L-arginyl-3-(2-naphthyl)-L-alaninamide 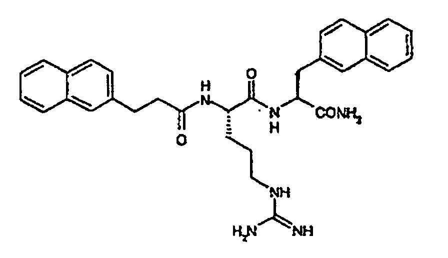
k. N²-[3-(1-Naphthyl)propionyl]-L-arginyl-3-(2-naphthyl)-L-alaninamide 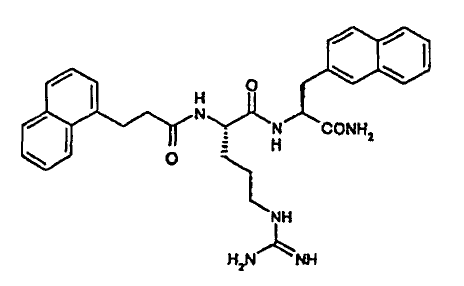
l. N²-[N-Acetyl-3-(3-benzothienyl)-L-alanyl]-L-arginyl-3-(2-naphthyl)-L-alaninamide 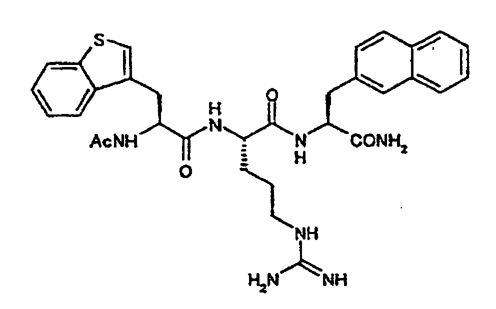
m. N²-[N-Acetyl-3-(3-benzothienyl)-L-alanyl]-L-arginyl-3-(2-naphthyl)-D-alaninamide 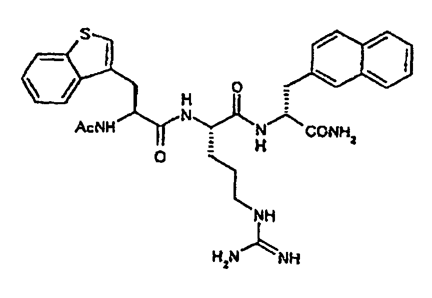
n. N²-[1-Naphthalenesulfonyl]-L-arginyl-3-(3-benzothienyl)-D-alaninamide

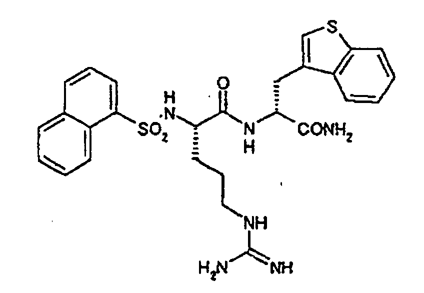

The compounds of Formula [1] can be prepared by the following general preparation method (in the following reaction schemes, Ar¹, Ar², Y¹, Y² and Q are as defined above; X is a hydroxyl group, a chlorine atom, a bromine atom or an iodine atom; P¹ is an ordinary amino-protective group such as a t-butoxycarbonyl group or a benzyloxycarbonyl group; P² and P³ are each an ordinary guanidino-protective group such as a t-butoxycarbonyl group, a benzyloxycarbonyl group, a nitro group, a tosyl group or a 2,2,5,7,8-pentamethylchroman-6-sulfonyl group).

### [General preparation method]

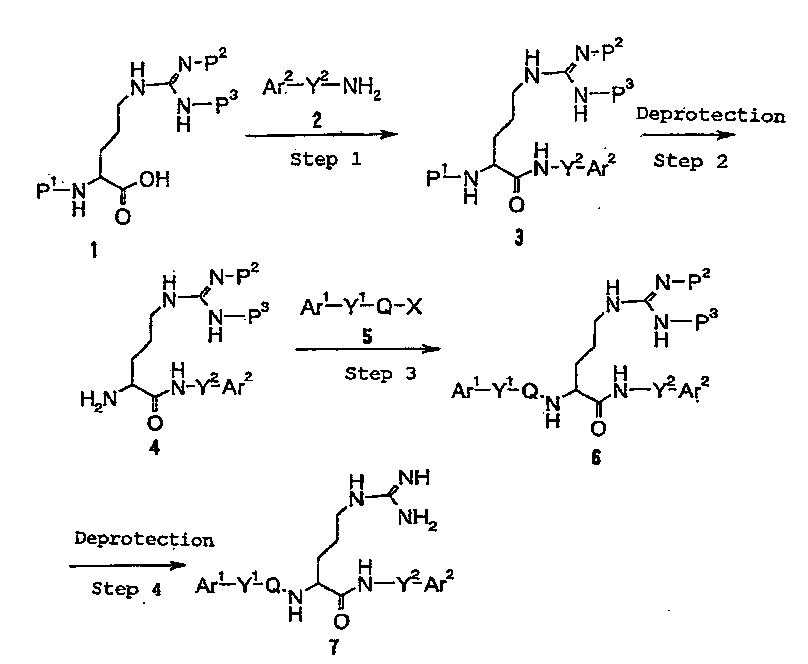

### [Step 1]

Compound (1) can be coupled with compound (2) in the presence or absence of a base in an inert solvent to convert to compound (3).

The base includes, for example, organic amines (e.g., triethylamine, diisopropylethylamine, pyridine and N-methylmorpholine) and inorganic bases (e.g., potassium carbonate and sodium bicarbonate). The coupling includes, for example, an amidation via an acid halide(e.g., an acid chloride and an acid bromide), an amidation via a mixed acid anhydride using ethyl chlorocarbonate, isobutyl chlorocarbonate, etc., and an amidation using a coupling agent such as 1-(3,3-dimethylaminopropyl)-3-ethylcarbodiimide, 1,3-dicyclohexylcarbodiimide, diphenylphosphoryl azide, diethyl cyanophosphate or carbonylimidazole. The inert solvent include, for example, alcohols (e.g., methanol and ethanol), ethers (e.g., diethyl ether and tetrahydrofuran), hydrocarbons (e.g., toluene and benzene), halogenated carbonaceous solvents (e.g., chloroform and dichloromethane), dimethylformamide, acetonitrile, water and a mixed solvent thereof.

### [Step 2]

Compound (3) can be deprotected in the presence or absence of an acid in an inert solvent to give compound (4). The deprotection of compound (3) can be carried out using the method described in Protective Groups in Organic Synthesis, by Theodora W. Greene and Peter G. M. Wuts.

### [Step 3]

Compound (4) can be coupled with compound (5) in the presence or absence of a base in an inert solvent to convert to compound (6). In case where Y¹ includes a protected amino group, acylation of the amino group can be carried out after deprotection in the presence or absence of a base in an inert solvent.

The base includes, for example, organic amines (e.g., triethylamine, diisopropylethylamine, pyridine and N-methylmorpholine) and inorganic bases (e.g., potassium carbonate and sodium bicarbonate). The coupling includes, for example, an amidation via an acid halide(e.g., an acid chloride and an acid bromide), an amidation via a mixed acid anhydride using ethyl chlorocarbonate, isobutyl chlorocarbonate, etc., and an amidation using a coupling agent such as 1-(3,3-dimethylaminopropyl)-3-ethylcarbodiimide, 1,3-dicyclohexylcarbodiimide, diphenylphosphoryl azide, diethyl cyanophosphate or carbonylimidazole. The inert solvent include, for example, alcohols (e.g., methanol and ethanol), ethers (e.g., diethyl ether and tetrahydrofuran), hydrocarbons (e.g., toluene and benzene), halogenated carbonaceous solvents (e.g., chloroform and dichloromethane), dimethylformamide, acetonitrile, water and a mixed solvent thereof. The protected amino group is a protected amino group described in Protective Groups in Organic Synthesis, by Theodora W. Greene and Peter G. M. Wuts; and examples of which are a t-butoxycarbonylamino group and a benzyloxycarbonylamino group. The deprotection is a deprotection of an amino group carried out according to the method described in Protective Groups in Organic Synthesis, by Theodora W. Greene and Peter G. M. Wuts. The acylation includes, for example, an acylation via an acid halide(e.g., an acid chloride and an acid bromide), an acylation using an acid anhydride (e.g., acetic anhydride), an acylation via a mixed acid anhydride using ethyl chlorocarbonate, isobutyl chlorocarbonate, etc., and an acylation using a coupling agent (e.g., 1-(3,3-dimethylaminopropyl)-3-ethylcarbodiimide, 1,3-dicyclohexylcarbodiimide, diphenylphosphoryl azide, diethyl cyanophosphate and carbonylimidazole).

### [Step 4]

Deprotection of a guanidino group can be carried out in the presence or absence of an acid in an inert solvent to give compound (6) of the present invention.

The deprotection of the guanidino group of the compound can be carried out using the method described in Protective Groups in Organic Synthesis, by Theodora W. Greene and Peter G. M. Wuts.

The dose of the compound according to the present invention, when used as a ligand for MC₄ receptor for the treatment of adult human, may range from 1 to 2000 mg per day, in a single portion or several divided portions. This dose can be suitably increased or decreased depending on application and age, body weight and conditions of the patient.

The compounds according to the present invention can be administered orally or parenterally, and the dosage forms thereof are, for example, tablets, capsules, granules, fine-powders, powders, troches, ointments, creams, emulsions, suspensions, suppositories, injections and nasal administration preparations, all of which can be prepared by conventional preparation techniques (e.g., the method defined in Japanese Pharmacopoeia, 14th edition). These dosage forms can be suitably chosen according to conditions and age of the patient and the purpose of therapy. For the productions of these forms, there can be used conventional excipients (e.g., crystalline cellulose, starches, lactose and mannitol), binders (e.g., hydroxypropylcellulose and polyvinyl pyrrolidone), lubricants (e.g., magnesium stearate and talc), disintegrators (e.g., carboxymethylcellulose calcium), etc.

### BEST MODE FOR CARRYING OUT THE INVENTION

The present invention is illustrated in more detail with reference to the following examples; however, the present invention is in no way limited to these examples (in the following formulae, Boc is a t-butoxycarbonyl group, Z is a benzyloxycarbonyl group and Ac is an acetyl group).

### Example 1 [Synthesis of compound 224 in Table 1]

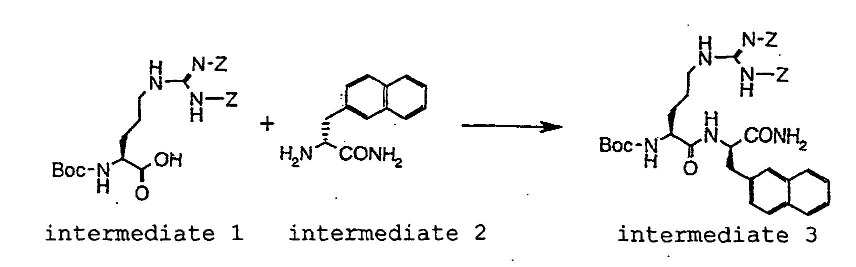

(1) In 20 mL of dimethylformamide were dissolved 2.16 g of intermediate 1, 1.00 g of intermediate 2, 0.92 g of 1-hydroxybenzotriazole monohydrate and 0.42 g of N-methylmorpholine, and then 0.96 g of 1-(3,3-dimethylaminopropyl)-3-ethylcarbodiimide hydrochloride was added with ice cooling. The temperature was slowly elevated to room temperature, followed by stirring for 3 days. The reaction solution was poured into a mixed solvent of ethyl acetate and water and, after separation of the solution, the organic layer was washed with 5 % aqueous potassium hydrogensulfate solution, a saturated aqueous sodium bicarbonate solution and a saturated aqueous sodium chloride solution, successively. After drying over anhydrous sodium sulfate, the drying agent was removed by filtration, followed by concentration under reduced pressure. The resulting crystals were recrystallized from ethyl acetate to give 2.27 g of intermediate 3. 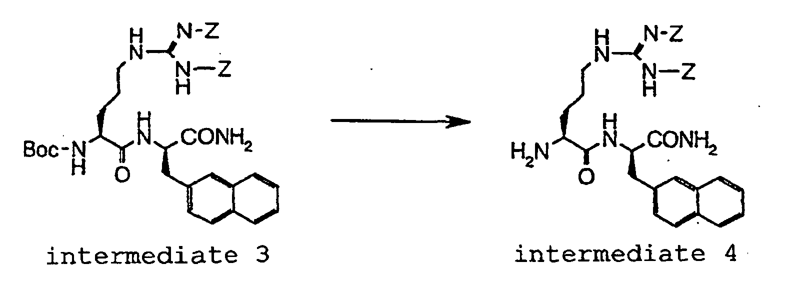
(2) In 15 mL of methylene chloride was dissolved 1.50 g of intermediate 3 obtained in (1), and 15 mL of trifluoroacetic acid was added thereto, followed by stirring at room temperature for 2 hours. The reaction solution was concentrated under reduced pressure, and a saturated aqueous sodium bicarbonate solution was poured, followed by extraction with chloroform. The organic layer was dried over anhydrous sodium sulfate, and the drying agent was removed by filtration, followed by concentration under reduced pressure to give a crude intermediate 4, which was then used for the next reaction without purification. 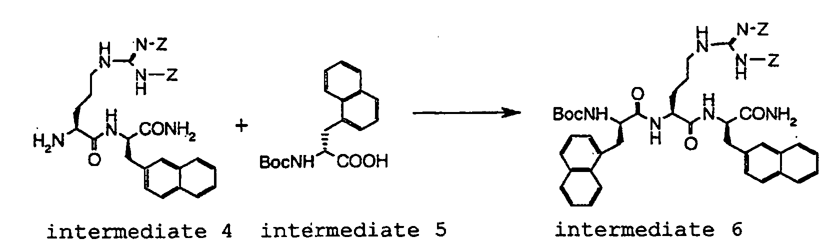
(3) In 15 mL of dimethylformamide were dissolved 0.42 g of intermediate 4 obtained in (2), 0.24 g of intermediate 5 and 0.16 g of 1-hydroxybenzotriazole monohydrate, and then 0.16 g of 1-(3,3-dimethylaminopropyl)-3-ethylcarbodiimide hydrochloride was added with ice cooling. The temperature was slowly elevated to room temperature, followed by stirring overnight. The reaction solution was poured into a mixed solvent of ethyl acetate and water and, after separation of the solution, the organic layer was washed with 5 % aqueous potassium hydrogensulfate solution, a saturated aqueous sodium bicarbonate solution and a saturated aqueous sodium chloride solution, successively. After drying over anhydrous sodium sulfate, the drying agent was removed by filtration, followed by concentration under reduced pressure. The residue was crystallized from ethyl acetate to give 0.43 g of intermediate 6. 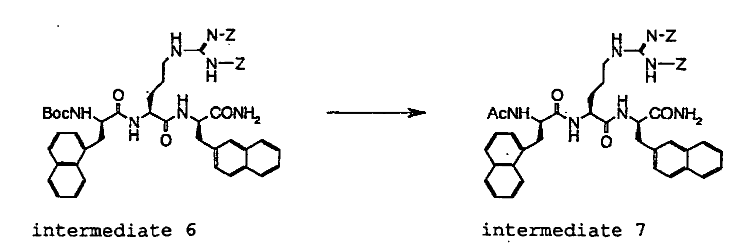
(4) In 5 mL of methylene chloride was dissolved 0.40 g of intermediate 6 obtained in (3), and 5 mL of trifluoroacetic acid was added thereto, followed by stirring at room temperature for 2 hours. The reaction solution was concentrated under reduced pressure, and a saturated aqueous sodium bicarbonate solution was poured, followed by extraction with chloroform. The organic layer was dried over anhydrous sodium sulfate, and the drying agent was removed by filtration, followed by concentration under reduced pressure. The residue was dissolved in 3 mL of methylene chloride, and 1 mL of a methylene chloride solution of 48 mg of acetic anhydride and 37 mg of pyridine was added, followed by stirring at room temperature for 2 hours. The reaction solution was concentrated under reduced pressure and, after pouring ethyl acetate, washed with water, 5 % aqueous potassium hydrogensulfate solution, a saturated aqueous sodium bicarbonate solution and a saturated aqueous sodium chloride solution, successively. After drying over anhydrous sodium sulfate, the drying agent was removed by filtration, followed by concentration under reduced pressure. The residue was crystallized from ethyl acetate to give 0.28 g of intermediate 7. 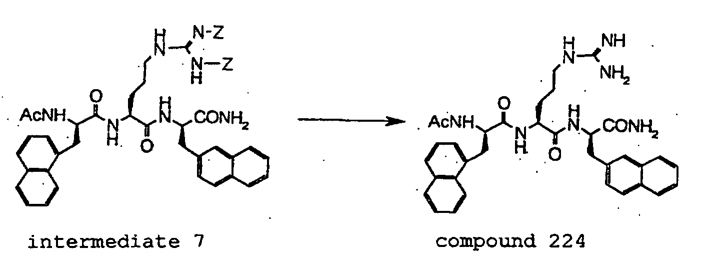
(5) In 10 mL of methanol was dissolved 0.28 g of intermediate 7 obtained in (4), and 100 mg of 20 % palladium hydroxide-activated carbon was added thereto, followed by stirring under a hydrogen atmosphere for 2 days. The reaction solution was filtered through Celite to remove the solid, and concentrated under reduced pressure. The residue was dissolved in 5 mL of methanol, 0.10 mL of 4M hydrogen chloride - ethyl acetate solution was added thereto and, after concentration under reduced pressure, solidification in ethyl acetate gave 0.18 g of hydrochloride of compound 224.

The structures and physical property data of the present compound and the compounds prepared similarly are shown in Table 1.

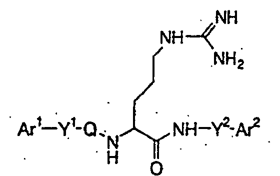

**Table 1**

| Comp.No | Ar¹-Y¹ | Q | Y²-Ar² | (M+1)^{+*1} |
|---|---|---|---|---|
| 001 | form(1) | CO | form(a) | 501.3 |
| 002 | form(1) | CO | form(b) | 501.3 |
| 003 | form(1) | CO | form(c) | 515.3 |
| 004 | form(1) | CO | form(d) | 515.3 |
| 005 | form(1) | CO | form(e) | 551.3 |
| 006 | form(1) | CO | form(f) | 551.3 |
| 007 | form(1) | CO | form(g) | 551.3 |
| 008 | form(1) | CO | form(h) | 551.3 |
| 009 | form(1) | CO | form(i) | 502.3 |
| 010 | form(1) | CO | form(j) | 502.3 |
| 011 | form(1) | CO | form(k) | 491.3 |
| 012 | form(1) | CO | form(l) | 491.3 |
| 013 | form(2) | CO | form(a) | 501.3 |
| 014 | form(2) | CO | form(b) | 501.3 |
| 015 | form(2) | CO | form(c) | 515.3 |
| 016 | form(2) | CO | form(d) | 515.3 |
| 017 | form(2) | CO | form(e) | 551.3 |
| 018 | form(2) | CO | form(f) | 551.3 |
| 019 | form(2) | CO | form(g) | 551.3 |
| 020 | form(2) | CO | form(h) | 551.3 |
| 021 | form(2) | CO | form(i) | 502.3 |
| 022 | form(2) | CO | form(j) | 502.3 |
| 023 | form(2) | CO | form(k) | 491.3 |
| 024 | form(2) | CO | form(l) | 491.3 |
| 025 | form(3) | CO | form(a) | 503.3 |
| 026 | form(3) | CO | form(b) | 503.3 |
| 027 | form(3) | CO | form(c) | 517.3 |
| 028 | form(3) | CO | form(d) | 517.3 |
| 029 | form(3) | CO | form(e) | 553.3 |
| 030 | form(3) | CO | form(f) | 553.3 |
| 031 | form(3) | CO | form(g) | 553.3 |
| 032 | form(3) | CO | form(h) | 553.3 |
| 033 | form(3) | CO | form(i) | 504.3 |
| 034 | form(3) | CO | form(j) | 504.3 |
| 035 | form(3) | CO | form(k) | 493.3 |
| 036 | form(3) | CO | form(l) | 493.3 |
| 037 | form(4) | CO | form(a) | 629.4 |
| 038 | form(4) | CO | form(b) | 629.4 |
| 039 | form(4) | CO | form(c) | 643.4 |
| 040 | form(4) | CO | form(d) | 643.4 |
| 041 | form(4) | CO | form(e) | 679.3 |
| 042 | form(4) | CO | form(f) | 679.3 |
| 043 | form(4) | CO | form(g) | 679.3 |
| 044 | form(4) | CO | form(h) | 679.3 |
| 045 | form(4) | CO | form(i) | 630.4 |
| 046 | form(4) | CO | form(j) | 630.4 |
| 047 | form(4) | CO | form(k) | 619.4 |
| 048 | form(4) | CO | form(l) | 619.4 |
| 049 | form(5) | CO | form(a) | 629.4 |
| 050 | form(5) | CO | form(b) | 629.4 |
| 051 | form(5) | CO | form(c) | 643.4 |
| 052 | form(5) | CO | form(d) | 643.4 |
| 053 | form(5) | CO | form(e) | 679.3 |
| 054 | form(5) | CO | form(f) | 679.3 |
| 055 | form(5) | CO | form(g) | 679.3 |
| 056 | form(5) | CO | form(h) | 679.3 |
| 057 | form(5) | CO | form(i) | 630.4 |
| 058 | form(5) | CO | form(j) | 630.4 |
| 059 | form(5) | CO | form(k) | 619.4 |
| 060 | form(5) | CO | form(l) | 619.4 |
| 061 | form(6) | CO | form(a) | 629.4 |
| 062 | form(6) | CO | form(b) | 629.4 |
| 063 | form(6) | CO | form(c) | 643.4 |
| 064 | form(6) | CO | form(d) | 643.4 |
| 065 | form(6) | CO | form(e) | 679.3 |
| 066 | form(6) | CO | form(f) | 679.3 |
| 067 | form(6) | CO | form(g) | 679.3 |
| 068 | form(6) | CO | form(h) | 679.3 |
| 069 | form(6) | CO | form(i) | 630.4 |
| 070 | form(6) | CO | form(j) | 630.4 |
| 071 | form(6) | CO | form(k) | 619.4 |
| 072 | form(6) | CO | form(l) | 619.4 |
| 073 | form(7) | SO₂ | form(a) | 511.2 |
| 074 | form(7) | SO₂ | form(b) | 511.2 |
| 075 | form(7) | SO₂ | form(c) | 525.3 |
| 076 | form(7) | SO₂ | form(d) | 525.3 |
| 077 | form(7) | SO₂ | form(e) | 561.3 |
| 078 | form(7) | SO₂ | form(f) | 561.3 |
| 079 | form(7) | SO₂ | form(g) | 561.3 |
| 080 | form(7) | SO₂ | form(h) | 561.3 |
| 081 | form(7) | SO₂ | form(i) | 512.2 |
| 082 | form(7) | SO₂ | form(j) | 512.3 |
| 083 | form(7) | SO₂ | form(k) | 501.2 |
| 084 | form(7) | SO₂ | form(l) | 501.2 |
| 085 | form(8) | SO₂ | form(a) | 554.3 |
| 086 | form(8) | SO₂ | form(b) | 554.3 |
| 087 | form(8) | SO₂ | form(c) | 568.3 |
| 088 | form(8) | SO₂ | form(d) | 568.3 |
| 089 | form(8) | SO₂ | form(e) | 604.3 |
| 090 | form(8) | SO₂ | form(f) | 604.3 |
| 091 | form(8) | SO₂ | form(g) | 604.3 |
| 092 | form(8) | SO₂ | form(h) | 604.3 |
| 093 | form(8) | SO₂ | form(i) | 555.3 |
| 094 | form(8) | SO₂ | form(j) | 555.3 |
| 095 | form(8) | SO₂ | form(k) | 544.3 |
| 096 | form(8) | SO₂ | form(l) | 544.3 |
| 097 | form(1) | CO | form(m) | 487.2 |
| 098 | form(1) | CO | form(n) | 487.2 |
| 099 | form(1) | CO | form(o) | 502.3 |
| 100 | form(1) | CO | form(p) | 502.3 |
| 101 | form(1) | CO | form(q) | 502.3 |
| 102 | form(1) | CO | form(r) | 557.2 |
| 103 | form(1) | CO | form(s) | 577.2 |
| 104 | form(1) | CO | form(t) | 577.2 |
| 105 | form(2) | CO | form(m) | 487.2 |
| 106 | form(2) | CO | form(n) | 487.3 |
| 107 | form(2) | CO | form(o) | 502.3 |
| 108 | form(2) | CO | form(p) | 502.3 |
| 109 | form(2) | CO | form(q) | 502.3 |
| 110 | form(2) | CO | form(r) | 557.2 |
| 111 | form(2) | CO | form(s) | 577.2 |
| 112 | form(2) | CO | form(t) | 577.2 |
| 113 | form(3) | CO | form(m) | 489.3 |
| 114 | form(3) | CO | form(n) | 489.3 |
| 115 | form(3) | CO | form(o) | 504.3 |
| 116 | form(3) | CO | form(p) | 504.3 |
| 117 | form(3) | CO | form(q) | 504.3 |
| 118 | form(3) | CO | form(r) | 559.2 |
| 119 | form(3) | CO | form(s) | 579.3 |
| 120 | form(3) | CO | form(t) | 579.3 |
| 121 | form(4) | CO | form(m) | 615.3 |
| 122 | form(4) | CO | form(n) | 615.3 |
| 123 | form(4) | CO | form(o) | 630.4 |
| 124 | form(4) | CO | form(p) | 630.3 |
| 125 | form(4) | CO | form(q) | 630.3 |
| 126 | form(4) | CO | form(r) | 685.3 |
| 127 | form(4) | CO | form(s) | 705.3 |
| 128 | form(4) | CO | form(t) | 705.3 |
| 129 | form(5) | CO | form(m) | 615.3 |
| 130 | form(5) | CO | form(n) | 615.3 |
| 131 | form(5) | CO | form(o) | 630.3 |
| 132 | form(5) | CO | form(p) | 630.3 |
| 133 | form(5) | CO | form(q) | 630.4 |
| 134 | form(5) | CO | form(r) | 685.2 |
| 135 | form(5) | CO | form(s) | 705.3 |
| 136 | form(5) | CO | form(t) | 705.3 |
| 137 | form(6) | CO | form(m) | 615.3 |
| 138 | form(6) | CO | form(n) | 615.3 |
| 139 | form(6) | CO | form(o) | 630.3 |
| 140 | form(6) | CO | form(p) | 630.3 |
| 141 | form(6) | CO | form(q) | 630.3 |
| 142 | form(6) | CO | form(r) | 685.2 |
| 143 | form(6) | CO | form(s) | 705.3 |
| 144 | form(6) | CO | form(t) | 705.3 |
| 145 | form(7) | SO₂ | form(m) | 497.2 |
| 146 | form(7) | SO₂ | form(n) | 497.2 |
| 147 | form(7) | SO₂ | form(p) | 512.2 |
| 148 | form(7) | SO₂ | form(r) | 567.1 |
| 149 | form(7) | SO₂ | form(s) | 587.2 |
| 150 | form(7) | SO₂ | form(t) | 587.2 |
| 151 | form(8) | SO₂ | form(m) | 540.3 |
| 152 | form(8) | SO₂ | form(n) | 540.3 |
| 153 | form(8) | SO₂ | form(p) | 555.2 |
| 154 | form(8) | SO₂ | form(r) | 610.2 |
| 155 | form(8) | SO₂ | form(s) | 630.3 |
| 156 | form(8) | SO₂ | form(t) | 630.3 |
| 157 | form(9) | CO | form(a) | 510.3 |
| 158 | form(9) | CO | form(b) | 510.3 |
| 159 | form(9) | CO | form(c) | 524.3 |
| 160 | form(9) | CO | form(d) | 524.3 |
| 161 | form(9) | CO | form(e) | 560.3 |
| 162 | form(9) | CO | form(f) | 560.3 |
| 163 | form(9) | CO | form(g) | 560.3 |
| 164 | form(9) | CO | form(h) | 560.3 |
| 165 | form(9) | CO | form(i) | 511.3 |
| 166 | form(9) | CO | form(j) | 511.3 |
| 167 | form(9) | CO | form(k) | 500.3 |
| 168 | form(9) | CO | form(l) | 500.3 |
| 169 | form(10) | CO | form(a) | 510.3 |
| 170 | form(10) | CO | form(b) | 510.3 |
| 171 | form(10) | CO | form(c) | 524.3 |
| 172 | form(10) | CO | form(d) | 524.3 |
| 173 | form(10) | CO | form(e) | 560.3 |
| 174 | form(10) | CO | form(f) | 560.3 |
| 175 | form(10) | CO | form(g) | 560.3 |
| 176 | form(10) | CO | form(h) | 560.3 |
| 177 | form(10) | CO | form(i) | 511.3 |
| 178 | form(10) | CO | form(j) | 511.3 |
| 179 | form(10) | CO | form(k) | 500.3 |
| 180 | form(10) | CO | form(l) | 500.3 |
| 181 | form(11) | CO | form(a) | 524.3 |
| 182 | form(11) | CO | form(b) | 524.3 |
| 183 | form(11) | CO | form(c) | 538.3 |
| 184 | form(11) | CO | form(d) | 538.3 |
| 185 | form(11) | CO | form(e) | 574.3 |
| 186 | form(11) | CO | form(f) | 574.3 |
| 187 | form(11) | CO | form(g) | 574.3 |
| 188 | form(11) | CO | form(h) | 574.3 |
| 189 | form(11) | CO | form(i) | 525.3 |
| 190 | form(11) | CO | form(j) | 525.3 |
| 191 | form(11) | CO | form(k) | 514.3 |
| 192 | form(11) | CO | form(l) | 514.3 |
| 193 | form(12) | CO | form(a) | 524.3 |
| 194 | form(12) | CO | form(b) | 524.3 |
| 195 | form(12) | CO | form(c) | 538.3 |
| 196 | form(12) | CO | form(d) | 538.3 |
| 197 | form(12) | CO | form(e) | 574.3 |
| 198 | form(12) | CO | form(f) | 574.3 |
| 199 | form(12) | CO | form(g) | 574.3 |
| 200 | form(12) | CO | form(h) | 574.3 |
| 201 | form(12) | CO | form(i) | 525.3 |
| 202 | form(12) | CO | form(j) | 525.3 |
| 203 | form(12) | CO | form(k) | 514.3 |
| 204 | form(12) | CO | form(l) | 514.3 |
| 205 | form(13) | CO | form(a) | 560.3 |
| 206 | form(13) | CO | form(b) | 560.3 |
| 207 | form(13) | CO | form(c) | 574.3 |
| 208 | form(13) | CO | form(d) | 574.3 |
| 209 | form(13) | CO | form(e) | 610.3 |
| 210 | form(13) | CO | form(f) | 610.3 |
| 211 | form(13) | CO | form(g) | 610.3 |
| 212 | form(13) | CO | form(h) | 610.3 |
| 213 | form(13) | CO | form(i) | 561.3 |
| 214 | form(13) | CO | form(j) | 561.3 |
| 215 | form(13) | CO | form(k) | 550.3 |
| 216 | form(13) | CO | form(l) | 550.3 |
| 217 | form(14) | CO | form(a) | 560.3 |
| 218 | form(14) | CO | form(b) | 560.3 |
| 219 | form(14) | CO | form(c) | 574.3 |
| 220 | form(14) | CO | form(d) | 574.3 |
| 221 | form(14) | CO | form(e) | 610.3 |
| 222 | form(14) | CO | form(f) | 610.3 |
| 223 | form(14) | CO | form(g) | 610.3 |
| 224 | form(14) | CO | form(h) | 610.3 |
| 225 | form(14) | CO | form(i) | 561.3 |
| 226 | form(14) | CO | form(j) | 561.3 |
| 227 | form(14) | CO | form(k) | 550.3 |
| 228 | form(14) | CO | form(l) | 550.3 |
| 229 | form(15) | CO | form(a) | 560.3 |
| 230 | form(15) | CO | form(b) | 560.3 |
| 231 | form(15) | CO | form(c) | 574.3 |
| 232 | form(15) | CO | form(d) | 574.3 |
| 233 | form(15) | CO | form(e) | 610.3 |
| 234 | form(15) | CO | form(f) | 610.3 |
| 235 | form(15) | CO | form(g) | 610.3 |
| 236 | form(15) | CO | form(h) | 610.3 |
| 237 | form(15) | CO | form(i) | 561.3 |
| 238 | form(15) | CO | form(j) | 561.3 |
| 239 | form(15) | CO | form(k) | 550.3 |
| 240 | form(15) | CO | form(l) | 550.3 |
| 241 | form(16) | CO | form(a) | 560.3 |
| 242 | form(16) | CO | form(b) | 560.3 |
| 243 | form(16) | CO | form(c) | 574.3 |
| 244 | form(16) | CO | form(d) | 574.3 |
| 245 | form(16) | CO | form(e) | 610.3 |
| 246 | form(16) | CO | form(f) | 610.3 |
| 247 | form(16) | CO | form(g) | 610.3 |
| 248 | form(16) | CO | form(h) | 610.3 |
| 249 | form(16) | CO | form(i) | 561.3 |
| 250 | form(16) | CO | form(j) | 561.3 |
| 251 | form(16) | CO | form(k) | 550.3 |
| 252 | form(16) | CO | form(l) | 550.3 |
| 253 | form(9) | CO | form(m) | 496.3 |
| 254 | form(9) | CO | form(n) | 496.3 |
| 255 | form(9) | CO | form(o) | 511.3 |
| 256 | form(9) | CO | form(p) | 511.3 |
| 257 | form(9) | CO | form(q) | 511.3 |
| 258 | form(9) | CO | form(r) | 566.2 |
| 259 | form(9) | CO | form(s) | 586.3 |
| 260 | form(9) | CO | form(t) | 586.3 |
| 261 | form(10) | CO | form(m) | 496.3 |
| 262 | form(10) | CO | form(n) | 496.3 |
| 263 | form(10) | CO | form(o) | 511.3 |
| 264 | form(10) | CO | form(p) | 511.3 |
| 265 | form(10) | CO | form(q) | 511.3 |
| 266 | form(10) | CO | form(r) | 566.2 |
| 267 | form(10) | CO | form(s) | 586.3 |
| 268 | form(10) | CO | form(t) | 586.3 |
| 269 | form(11) | CO | form(m) | 510.3 |
| 270 | form(11) | CO | form(n) | 510.3 |
| 271 | form(11) | CO | form(o) | 525.3 |
| 272 | form(11) | CO | form(p) | 525.3 |
| 273 | form(11) | CO | form(q) | 525.3 |
| 274 | form(11) | CO | form(r) | 580.2 |
| 275 | form(11) | CO | form(s) | 600.3 |
| 276 | form(11) | CO | form(t) | 600.3 |
| 277 | form(12) | CO | form(m) | 510.3 |
| 278 | form(12) | CO | form(n) | 510.3 |
| 279 | form(12) | CO | form(o) | 525.3 |
| 280 | form(12) | CO | form(p) | 525.3 |
| 281 | form(12) | CO | form(q) | 525.3 |
| 282 | form(12) | CO | form(r) | 580.2 |
| 283 | form(12) | CO | form(s) | 600.3 |
| 284 | form(12) | CO | form(t) | 600.3 |
| 285 | form(13) | CO | form(m) | 546.3 |
| 286 | form(13) | CO | form(n) | 546.3 |
| 287 | form(13) | CO | form(o) | 561.3 |
| 288 | form(13) | CO | form(p) | 561.3 |
| 289 | form(13) | CO | form(q) | 561.3 |
| 290 | form(13) | CO | form(r) | 616.3 |
| 291 | form(13) | CO | form(s) | 636.4 |
| 292 | form(13) | CO | form(t) | 636.4 |
| 293 | form(14) | CO | form(m) | 546.3 |
| 294 | form(14) | CO | form(n) | 546.3 |
| 295 | form(14) | CO | form(o) | 561.3 |
| 296 | form(14) | CO | form(p) | 561.3 |
| 297 | form(14) | CO | form(q) | 561.3 |
| 298 | form(14) | CO | form(r) | 616.3 |
| 299 | form(14) | CO | form(s) | 616.4 |
| 300 | form(14) | CO | form(t) | 636.4 |
| 301 | form(15) | CO | form(m) | 546.3 |
| 302 | form(15) | CO | form(n) | 546.3 |
| 303 | form(15) | CO | form(o) | 561.2 |
| 304 | form(15) | CO | form(p) | 561.3 |
| 305 | form(15) | CO | form(q) | 561.3 |
| 306 | form(15) | CO | form(r) | 616.3 |
| 307 | form(15) | CO | form(s) | 636.4 |
| 308 | form(15) | CO | form(t) | 636.4 |
| 309 | form(16) | CO | form(m) | 546.3 |
| 310 | form(16) | CO | form(n) | 546.3 |
| 311 | form(16) | CO | form(o) | 561.3 |
| 312 | form(16) | CO | form(p) | 561.3 |
| 313 | form(16) | CO | form(q) | 561.3 |
| 314 | form(16) | CO | form(r) | 616.3 |
| 315 | form(16) | CO | form(s) | 636.4 |
| 316 | form(16) | CO | form(t) | 636.4 |
| 317 | form(17) | CO | form(a) | 511.3 |
| 318 | form(17) | CO | form(b) | 511.3 |
| 319 | form(17) | CO | form(c) | 525.3 |
| 320 | form(17) | CO | form(d) | 525.3 |
| 321 | form(17) | CO | form(e) | 561.3 |
| 322 | form(17) | CO | form(f) | 561.3 |
| 323 | form(17) | CO | form(g) | 561.3 |
| 324 | form(17) | CO | form(h) | 561.3 |
| 325 | form(17) | CO | form(i) | 512.3 |
| 326 | form(17) | CO | form(j) | 512.3 |
| 327 | form(17) | CO | form(k) | 501.3 |
| 328 | form(17) | CO | form(l) | 501.3 |
| 329 | form(18) | CO | form(a) | 511.3 |
| 330 | form(18) | CO | form(b) | 511.3 |
| 331 | form(18) | CO | form(c) | 525.3 |
| 332 | form(18) | CO | form(d) | 525.3 |
| 333 | form(18) | CO | form(e) | 561.3 |
| 334 | form(18) | CO | form(f) | 561.3 |
| 335 | form(18) | CO | form(g) | 561.3 |
| 336 | form(18) | CO | form(h) | 561.3 |
| 337 | form(18) | CO | form(i) | 512.3 |
| 338 | form(18) | CO | form(j) | 512.3 |
| 339 | form(18) | CO | form(k) | 501.3 |
| 340 | form(18) | CO | form(l) | 501.3 |
| 341 | form(19) | CO | form(a) | 500.3 |
| 342 | form(19) | CO | form(b) | 500.3 |
| 343 | form(19) | CO | form(c) | 514.3 |
| 344 | form(19) | CO | form(d) | 514.3 |
| 345 | form(19) | CO | form(e) | 550.3 |
| 346 | form(19) | CO | form(f) | 550.3 |
| 347 | form(19) | CO | form(g) | 550.3 |
| 348 | form(19) | CO | form(h) | 550.3 |
| 349 | form(19) | CO | form(i) | 501.3 |
| 350 | form(19) | CO | form(j) | 501.3 |
| 351 | form(19) | CO | form(k) | 490.3 |
| 352 | form(19) | CO | form(l) | 490.3 |
| 353 | form(20) | CO | form(a) | 500.3 |
| 354 | form(20) | CO | form(b) | 500.3 |
| 355 | form(20) | CO | form(c) | 514.3 |
| 356 | form(20) | CO | form(d) | 514.3 |
| 357 | form(20) | CO | form(e) | 550.3 |
| 358 | form(20) | CO | form(f) | 550.3 |
| 359 | form(20) | CO | form(g) | 550.3 |
| 360 | form(20) | CO | form(h) | 550.3 |
| 361 | form(20) | CO | form(i) | 501.3 |
| 362 | form(20) | CO | form(j) | 501.3 |
| 363 | form(20) | CO | form(k) | 490.3 |
| 364 | form(20) | CO | form(l) | 490.3 |
| 365 | form(21) | CO | form(a) | 496.3 |
| 366 | form(21) | CO | form(b) | 496.3 |
| 367 | form(21) | CO | form(c) | 510.3 |
| 368 | form(21) | CO | form(d) | 510.3 |
| 369 | form(21) | CO | form(e) | 546.3 |
| 370 | form(21) | CO | form(f) | 546.3 |
| 371 | form(21) | CO | form(g) | 546.3 |
| 372 | form(21) | CO | form(h) | 546.3 |
| 373 | form(21) | CO | form(i) | 497.3 |
| 374 | form(21) | CO | form(j) | 497.3 |
| 375 | form(21) | CO | form(k) | 486.3 |
| 376 | form(21) | CO | form(l) | 486.3 |
| 377 | form(22) | CO | form(a) | 496.3 |
| 378 | form(22) | CO | form(b) | 496.3 |
| 379 | form(22) | CO | form(c) | 510.3 |
| 380 | form(22) | CO | form(d) | 510.3 |
| 381 | form(22) | CO | form(e) | 546.3 |
| 382 | form(22) | CO | form(f) | 546.3 |
| 383 | form(22) | CO | form(g) | 546.3 |
| 384 | form(22) | CO | form(h) | 546.3 |
| 385 | form(22) | CO | form(i) | 497.3 |
| 386 | form(22) | CO | form(j) | 497.3 |
| 387 | form(22) | CO | form(k) | 486.3 |
| 388 | form(22) | CO | form(l) | 486.3 |
| 389 | form(23) | CO | form(a) | 511.3 |
| 390 | form(23) | CO | form(b) | 511.3 |
| 391 | form(23) | CO | form(c) | 525.3 |
| 392 | form(23) | CO | form(d) | 525.3 |
| 393 | form(23) | CO | form(e) | 561.3 |
| 394 | form(23) | CO | form(f) | 561.3 |
| 395 | form(23) | CO | form(g) | 561.3 |
| 396 | form(23) | CO | form(h) | 561.3 |
| 397 | form(23) | CO | form(i) | 512.3 |
| 398 | form(23) | CO | form(j) | 512.3 |
| 399 | form(23) | CO | form(k) | 501.3 |
| 400 | form(23) | CO | form(l) | 501.3 |
| 401 | form(24) | CO | form(a) | 511.3 |
| 402 | form(24) | CO | form(b) | 511.3 |
| 403 | form(24) | CO | form(c) | 525.3 |
| 404 | form(24) | CO | form(d) | 525.3 |
| 405 | form(24) | CO | form(e) | 561.3 |
| 406 | form(24) | CO | form(f) | 561.3 |
| 407 | form(24) | CO | form(g) | 561.3 |
| 408 | form(24) | CO | form(h) | 561.3 |
| 409 | form(24) | CO | form(i) | 512.3 |
| 410 | form(24) | CO | form(j) | 512.3 |
| 411 | form(24) | CO | form(k) | 501.3 |
| 412 | form(24) | CO | form(l) | 501.3 |
| 413 | form(17) | CO | form(m) | 497.3 |
| 414 | form(17) | CO | form(n) | 497.3 |
| 415 | form(17) | CO | form(o) | 512.3 |
| 416 | form(17) | CO | form(p) | 512.3 |
| 417 | form(17) | CO | form(q) | 512.3 |
| 418 | form(17) | CO | form(r) | 567.2 |
| 419 | form(17) | CO | form(s) | 587.3 |
| 420 | form(17) | CO | form(t) | 587.3 |
| 421 | form(18) | CO | form(m) | 497.3 |
| 422 | form(18) | CO | form(n) | 497.3 |
| 423 | form(18) | CO | form(o) | 512.3 |
| 424 | form(18) | CO | form(p) | 512.3 |
| 425 | form(18) | CO | form(q) | 512.3 |
| 426 | form(18) | CO | form(r) | 567.2 |
| 427 | form(18) | CO | form(s) | 587.3 |
| 428 | form(18) | CO | form(t) | 587.3 |
| 429 | form(19) | CO | form(m) | 486.3 |
| 430 | form(19) | CO | form(n) | 486.3 |
| 431 | form(19) | CO | form(o) | 501.3 |
| 432 | form(19) | CO | form(p) | 501.3 |
| 433 | form(19) | CO | form(q) | 501.3 |
| 434 | form(19) | CO | form(r) | 556.2 |
| 435 | form(19) | CO | form(s) | 576.3 |
| 436 | form(19) | CO | form(t) | 576.3 |
| 437 | form(20) | CO | form(m) | 486.3 |
| 438 | form(20) | CO | form(n) | 486.3 |
| 439 | form(20) | CO | form(o) | 501.3 |
| 440 | form(20) | CO | form(p) | 501.3 |
| 441 | form(20) | CO | form(q) | 501.3 |
| 442 | form(20) | CO | form(r) | 556.2 |
| 443 | form(20) | CO | form(s) | 576.3 |
| 444 | form(20) | CO | form(t) | 576.3 |
| 445 | form(21) | CO | form(m) | 482.2 |
| 446 | form(21) | CO | form(n) | 482.2 |
| 447 | form(21) | CO | form(o) | 497.3 |
| 448 | form(21) | CO | form(p) | 497.3 |
| 449 | form(21) | CO | form(q) | 497.3 |
| 450 | form(21) | CO | form(r) | 552.2 |
| 451 | form(21) | CO | form(s) | 572.3 |
| 452 | form(21) | CO | form(t) | 572.3 |
| 453 | form(22) | CO | form(m) | 482.3 |
| 454 | form(22) | CO | form(n) | 482.3 |
| 455 | form(22) | CO | form(o) | 497.3 |
| 456 | form(22) | CO | form(p) | 497.3 |
| 457 | form(22) | CO | form(q) | 497.3 |
| 458 | form(22) | CO | form(r) | 552.2 |
| 459 | form(22) | CO | form(s) | 572.3 |
| 460 | form(22) | CO | form(t) | 572.3 |
| 461 | form(23) | CO | form(m) | 497.3 |
| 462 | form(23) | CO | form(n) | 497.3 |
| 463 | form(23) | CO | form(o) | 512.3 |
| 464 | form(23) | CO | form(p) | 512.3 |
| 465 | form(23) | CO | form(q) | 512.3 |
| 466 | form(23) | CO | form(r) | 567.2 |
| 467 | form(23) | CO | form(s) | 587.3 |
| 468 | form(23) | CO | form(t) | 587.3 |
| 469 | form(24) | CO | form(m) | 497.3 |
| 470 | form(24) | CO | form(n) | 497.3 |
| 471 | form(24) | CO | form(o) | 512.3 |
| 472 | form(24) | CO | form(p) | 512.3 |
| 473 | form(24) | CO | form(q) | 512.4 |
| 474 | form(24) | CO | form(r) | 567.2 |
| 475 | form(24) | CO | form(s) | 587.3 |
| 476 | form(24) | CO | form(t) | 587.3 |
| 477 | form(25) | CO | form(a) | 511.3 |
| 478 | form(25) | CO | form(b) | 511.3 |
| 479 | form(25) | CO | form(c) | 525.3 |
| 480 | form(25) | CO | form(d) | 525.3 |
| 481 | form(25) | CO | form(e) | 561.3 |
| 482 | form(25) | CO | form(f) | 561.3 |
| 483 | form(25) | CO | form(g) | 561.3 |
| 484 | form(25) | CO | form(h) | 561.3 |
| 485 | form(25) | CO | form(i) | 512.3 |
| 486 | form(25) | CO | form(j) | 512.3 |
| 487 | form(25) | CO | form(k) | 501.3 |
| 488 | form(25) | CO | form(l) | 501.3 |
| 489 | form(26) | CO | form(a) | 566.2 |
| 490 | form(26) | CO | form(b) | 566.2 |
| 491 | form(26) | CO | form(c) | 580.2 |
| 492 | form(26) | CO | form(d) | 580.2 |
| 493 | form(26) | CO | form(e) | 616.3 |
| 494 | form(26) | CO | form(f) | 616.3 |
| 495 | form(26) | CO | form(g) | 616.3 |
| 496 | form(26) | CO | form(h) | 616.3 |
| 497 | form(26) | CO | form(i) | 567.2 |
| 498 | form(26) | CO | form(j) | 567.2 |
| 499 | form(26) | CO | form(k) | 556.2 |
| 500 | form(26) | CO | form(l) | 556.2 |
| 501 | form(27) | CO | form(a) | 586.3 |
| 502 | form(27) | CO | form(b) | 586.3 |
| 503 | form(27) | CO | form(c) | 600.3 |
| 504 | form(27) | CO | form(d) | 600.3 |
| 505 | form(27) | CO | form(e) | 636.4 |
| 506 | form(27) | CO | form(f) | 636.4 |
| 507 | form(27) | CO | form(g) | 636.4 |
| 508 | form(27) | CO | form(h) | 636.4 |
| 509 | form(27) | CO | form(i) | 587.3 |
| 510 | form(27) | CO | form(j) | 587.3 |
| 511 | form(27) | CO | form(k) | 576.3 |
| 512 | form(27) | CO | form(l) | 576.3 |
| 513 | form(28) | CO | form(a) | 586.3 |
| 514 | form(28) | CO | form(b) | 586.3 |
| 515 | form(28) | CO | form(c) | 600.3 |
| 516 | form(28) | CO | form(d) | 600.3 |
| 517 | form(28) | CO | form(e) | 636.4 |
| 518 | form(28) | CO | form(f) | 636.4 |
| 519 | form(28) | CO | form(g) | 636.3 |
| 520 | form(28) | CO | form(h) | 636.4 |
| 521 | form(28) | CO | form(i) | 587.3 |
| 522 | form(28) | CO | form(j) | 587.3 |
| 523 | form(28) | CO | form(k) | 576.2 |
| 524 | form(28) | CO | form(l) | 576.3 |
| 525 | form(25) | CO | form(m) | 497.3 |
| 526 | form(25) | CO | form(n) | 497.3 |
| 527 | form(25) | CO | form(o) | 512.3 |
| 528 | form(25) | CO | form(p) | 512.3 |
| 529 | form(25) | CO | form(q) | 512.3 |
| 530 | form(25) | CO | form(r) | 567.2 |
| 531 | form(25) | CO | form(s) | 587.3 |
| 532 | form(25) | CO | form(t) | 587.3 |
| 533 | form(26) | CO | form(m) | 552.2 |
| 534 | form(26) | CO | form(n) | 552.2 |
| 535 | form(26) | CO | form(p) | 567.2 |
| 536 | form(26) | CO | form(q) | 567.2 |
| 537 | form(26) | CO | form(r) | 622.2 |
| 538 | form(26) | CO | form(s) | 642.3 |
| 539 | form(26) | CO | form(t) | 642.3 |
| 540 | form(27) | CO | form(m) | 572.3 |
| 541 | form(27) | CO | form(n) | 572.3 |
| 542 | form(27) | CO | form(o) | 587.2 |
| 543 | form(27) | CO | form(p) | 587.3 |
| 544 | form(27) | CO | form(q) | 587.3 |
| 545 | form(27) | CO | form(r) | 642.3 |
| 546 | form(27) | CO | form(s) | 662.3 |
| 547 | form(27) | CO | form(t) | 662.3 |
| 548 | form(28) | CO | form(m) | 572.2 |
| 549 | form(28) | CO | form(n) | 572.2 |
| 550 | form(28) | CO | form(o) | 587.3 |
| 551 | form(28) | CO | form(p) | 587.3 |
| 552 | form(28) | CO | form(q) | 587.3 |
| 553 | form(28) | CO | form(r) | 642.3 |
| 554 | form(28) | CO | form(s) | 662.3 |
| 555 | form(28) | CO | form(t) | 662.3 |
| 556 | form(29) | CO | form(u) | *2 |
| 557 | form(29) | CO | form(w) | *3 |
| 558 | form(29) | CO | form(x) | *4 |
| 559 | form(4) | CO | form(u) | *5 |

| | | | | |
|---|---|---|---|---|
| *1:ESIMS(Pos) | | | | |
| *2:HRMS(FAB) calcd for C₃₁H₃₅N₇O₃ 554.2880, found 554.2908 | | | | |
| *3:HRMS(FAB) calcd for C₃₃H₃₉N₇O₃ 582.3193, found 582.3195 | | | | |
| *4:HRMS(FAB) calcd for C₃₁H₃₆N₆O₃ 541.2927; found 541.2955 | | | | |
| *5:HRMS(FAB) calcd for C₄₀H₄₁N₇O₃ 668.3350, found 668.3358 | | | | |

Ar¹-Y¹-

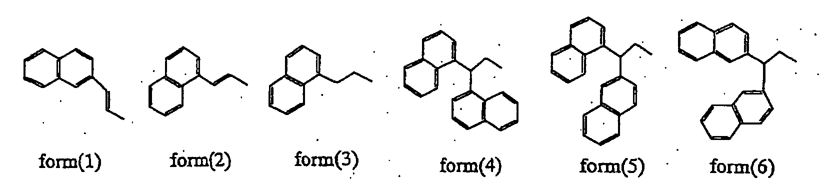

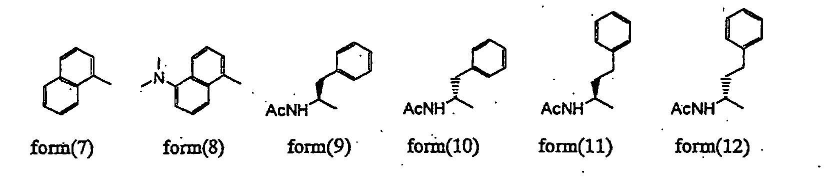

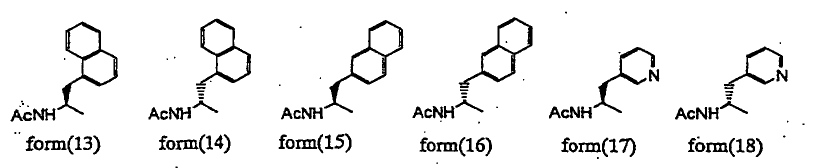

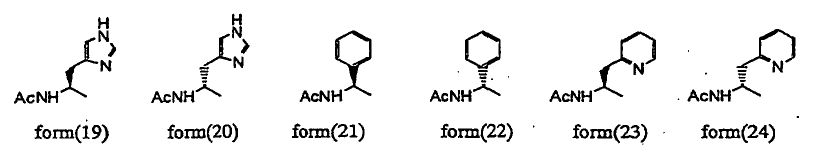

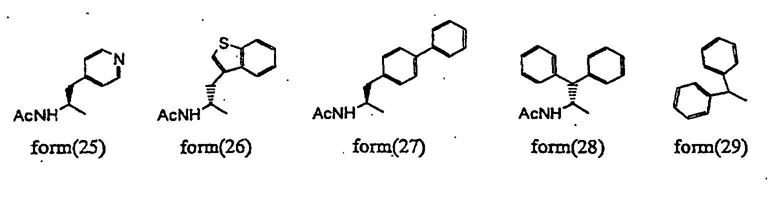

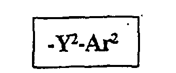

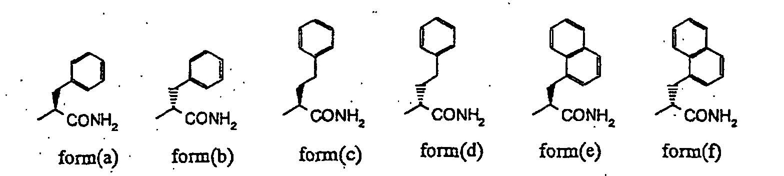

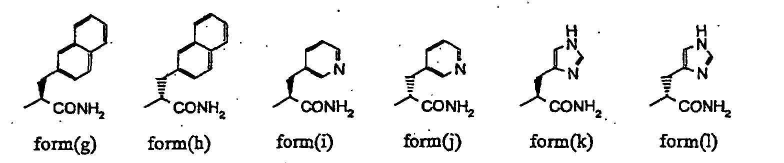

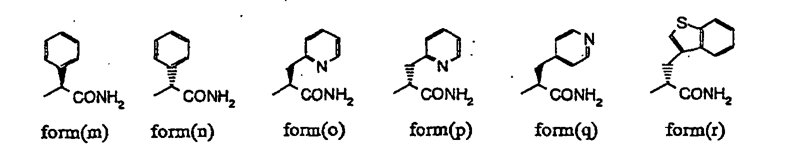

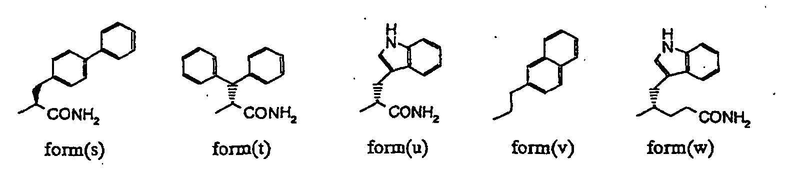

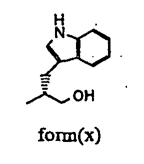

### Experiment 1 [MC₄ receptor Binding Assay]

MC₄ receptor binding assay was carried out according to the method described in Pharmacology & Toxicology, 79, 161-165, 1996. HEK-293 cell membranes expressing the human MC₄ receptor were purchased from Biolinks Co. The cell membranes were homogenized in a 50 mM Tris hydrochloric acid buffer solution (pH 7.4) containing 2 mM ethylenediamine tetraacetic acid, 10 mM calcium chloride and 100 µM phenylmethanesulfonylfluoride. The homogenate was centrifuged at 48,000 x g for 20 minutes at 4°C. The precipitate obtained by centrifugation was again homogenized in the same buffer solution, and the homogenate was centrifuged at 48,000 x g for 20 minutes at 4°C. This procedure was repeated twice. The precipitate was suspended in 50 mM Tris hydrochloric acid buffer solution (pH 7.4) containing 2 mM ethylenediamine tetraacetic acid, 10 mM calcium chloride, 100 µM phenylmethanesulfonylfluoride and 0.1 % bovine serum albumin to adjust to a protein concentration of 100 µg/ml to give a crude membrane preparation which was used for the binding assay. The crude membrane preparation (0.25 ml, 25 µg protein) was reacted with [¹²⁵I]Nle⁴-D-Phe⁷-α-MSH (final concentration; 0.2 nM) at 25°C for 120 minutes. After the completion of the reaction, the reaction solution was filtered under suction on GF/C glass filter presoaked for 2 hours in 50 mM Tris hydrochloric acid buffer solution (pH 7.4) containing 0.5 % bovine serum with the use of a cell harvester for receptor binding assay. The radioactivity on the filter paper was measured in a gamma-counter. The binding in the presence of 1 µM Nle⁴-D-Phe⁷-α-MSH was defined as non-specific binding. Specific binding was obtained by subtracting the non-specific binding from the total binding, which was the binding in the absence of 1 µM Nle⁴-D-Phe⁷-α-MSH. Test compound was dissolved in 100 % DMSO, and added simultaneously with [¹²⁵I]Nle⁴-D-Phe⁷-α-MSH to the membrane preparation. The IC₅₀ value was calculated from the inhibition curve in the concentration of 10⁻⁹ - 10⁻⁵.

As a result, for example, the IC₅₀ value of compound 224 was 690 nM.

### INDUSTRIAL APPLICABILITY

The compounds represented by Formula [1] or pharmaceutically acceptable salts thereof are useful as peptidergic ligands which have the affinity and specificity to MC₄ receptor and are useful as medicines.

## Claims

1. An arginine derivative represented by the formula: 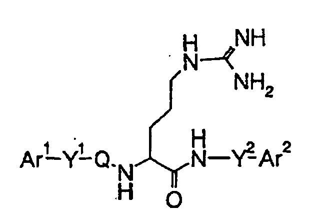 [wherein Ar¹ and Ar² may be the same or different, and are each a phenyl group, a substituted phenyl group, a naphthyl group, a substituted naphthyl group or a heteroaromatic ring group containing one or more of nitrogen, oxygen and sulfur atoms; Y¹ is a C₁₋₅ alkylene group, a C₂₋₅ alkenylene group or a single bond; and the C₁₋₅ alkylene group optionally contains a carbon atom substituted with a phenyl group, a substituted phenyl group, a naphthyl group, a substituted naphthyl group or a C₁₋₁₀ acylamino group; Q is a carbonyl group or a sulfonyl group; Y² is a C₁₋₅ alkylene group; the C₁₋₅ alkylene group optionally contains a carbon atom substituted with a phenyl group, a substituted phenyl group, a naphthyl group, a substituted naphthyl group, a hydroxyl group, a carbamoyl group, a mono-C₁₋₅ alkylamide group or a di-C₁₋₅ alkylamide group], or a pharmaceutically acceptable salt thereof.

2. The arginine derivative or a pharmaceutically acceptable salt thereof according to Claim 1, wherein, the substituted phenyl group is a phenyl group substituted with 1 to 3 substituents selected arbitrarily from the group consisting of a C₁₋₅ alkyl group, a C₁₋₅ alkoxy group, an aralkyloxy group, a hydroxyl group, a halogen atom, a nitro group, an amino group, a mono-C₁₋₅ alkylamino group, a di-C₁₋₅ alkylamino group, a trifluoromethyl group and a phenyl group; the substituted naphthyl group is a naphthyl group substituted with 1 to 3 substituents selected arbitrarily from the group consisting of a C₁₋₅ alkyl group, a C₁₋₅ alkoxy group, an aralkyloxy group, a halogen atom, a nitro group, an amino group, a mono-C₁₋₅ alkylamino group, a di-C₁₋₅ alkylamino group, a trifluoromethyl group and a phenyl group; and the heteroaromatic ring group is a monocyclic or dicyclic aromatic ring group which contains one or more of nitrogen, oxygen and sulfur atoms.

3. The arginine derivative or a pharmaceutically acceptable salt thereof according to Claim 1, wherein, Ar¹ and Ar² may be the same or different, and are each a phenyl group, a naphthyl group or a 3-benzothienyl group, Y¹ is a C₁₋₂ alkylene group or a C₁₋₂ alkylene group substituted with one acetoamino group; Q is a carbonyl group or a sulfonyl group; Y² is a C₁₋₂ alkylene group or a C₁₋₂ alkylene group substituted with one carbamoyl group.

4. The arginine derivative or a pharmaceutically acceptable salt thereof according to Claim 1, which is selected from the following a - n:
a. N²-[N-Acetyl-3-(2-naphthyl)-D-alanyl]-L-arginyl-3-(2-naphthyl)-D-alaninamide,
b. N²-[N-Acetyl-3-(2-naphthyl)-D-alanyl]-L-arginyl-3-(2-naphthyl)-L-alaninamide,
c. N²-[N-Acetyl-3-(2-naphthyl)-D-alanyl]-N-[2-(2-naphthyl)ethyl]-L-argininamide,
d. N²-[N-Acetyl-3-(1-naphthyl)-D-alanyl]-L-arginyl-3-(2-naphthyl)-D-alaninamide,
e. N²-[N-Acetyl-3-(1-naphthyl)-D-alanyl]-L-arginyl-3-(2-naphthyl)-L-alaninamide,
f. N²-[N-Acetyl-3-(1-naphthyl)-D-alanyl]-L-arginyl-3-(1-naphthyl)-L-alaninamide,
g. N²-[N-Acetyl-D-phenylalanyl]-L-arginyl-3-(2-naphthyl)-D-alaninamide,
h. N²-[N-Acetyl-D-phenylalanyl]-L-arginyl-3-(2-naphthyl)-L-alaninamide,
i. N²-[N-Acetyl-D-phenylalanyl]-N-[2-(2-naphthyl)ethyl]-L-argininamide,
j. N²-[3-(2-Naphthyl)propionyl]-L-arginyl-3-(2-naphthyl)-L-alaninamide,
k. N²-[3-(1-Naphthyl)propionyl]-L-arginyl-3-(2-naphthyl)-L-alaninamide,
l. N²-[N-Acetyl-3-(3-benzothienyl)-L-alanyl]-L-arginyl-3-(2-naphthyl)-L-alaninamide,
m. N²-[N-Acetyl-3-(3-benzothienyl)-L-alanyl]-L-arginyl-3-(2-naphthyl)-D-alaninamide, and
n. N²-[1-Naphthalenesulfonyl]-L-arginyl-3-(3-benzothienyl)-D-alaninamide.

5. A medicine comprising the arginine derivative or a pharmaceutically acceptable salt thereof according to any one of Claims 1 to 4.

6. Use of the arginine derivative or a pharmaceutically acceptable salt thereof according to any one of Claims 1 to 4 as a ligand for MC₄ receptor.
